Europäisches Patentamt

European Patent Office

Office européen des brevets

(19)

(11) Publication number: **0 231 759 B1**

# EUROPEAN PATENT SPECIFICATION

(45) Date of publication of patent specification: **11.12.91**   (51) Int. Cl.⁵: **C07C 67/08**, C07H 13/06

(21) Application number: **87100197.0**

(22) Date of filing: **09.01.87**

(54) **Process for producing peracetylated sugar alcohols from sugar alcohols having at least four carbon atoms.**

(30) Priority: **16.01.86 DE 3601098**

(43) Date of publication of application:
**12.08.87 Bulletin 87/33**

(45) Publication of the grant of the patent:
**11.12.91 Bulletin 91/50**

(84) Designated Contracting States:
**AT BE CH DE ES FR GB IT LI NL SE**

(56) References cited:
**EP-A- 0 091 159**

(73) Proprietor: **CPC INTERNATIONAL INC.**
**International Plaza P.O. Box 8000**
**Englewood Cliffs New Jersey 07632(US)**

(72) Inventor: **Kutschker, Wolfram, Dr.**
**Friesenstrasse 8**
**WE-4150 Krefeld(DE)**
Inventor: **Feldmann, John, Dr.**
**Avenue du Général de Gaulle 49**
**B-1050 Bruxelles(BE)**
Inventor: **Koebernick, Hubert, Dr.**
**Grüner Dyk 85**
**WE-4150 Krefeld(DE)**

(74) Representative: **Lederer, Franz, Dr. et al**
**Lederer, Keller & Riederer Patentanwälte**
**Lucile-Grahn-Strasse 22**
**W-8000 München 80(DE)**

## Description

The invention relates to a process for producing peracetylated sugar alcohols from sugar alcohols having at least four carbon atoms by reaction with acetic anhydride at elevated temperatures in the presence of an alkali acetate catalyst.

Peracetylated sugar alcohols such as hexaacetyl sorbitol, and their use in activators or so-called "boosters", in certain bleaching agents of the oxygen type, i.e. alkali percarbonates, are known (DE-OS 25 10 184).

EP-A-91159 discloses a process for preparing sugar acetates comprising a first reaction stage wherein a sugar, selected from glucose and xylose, is catalytically reacted with acetic acid at a ratio of one mole of sugar to more than 2 moles of acetic acid to form a diacetylated product (glucose diacetate or xylose diacetate), followed by a second reaction stage wherein said diacetylated product is further catalytically reacted with excess acetic anhydride to form the respective glucose penta-acetate or xylose tetra-acetate.

The production of peracetylated sugar alcohols is possible by a number of esterification methods such as reacting the sugar alcohol with ketene, with acetyl chloride in the presence of a base such as sodium hydroxide as catalyst, or with acetic anhydride in the presence of pyridine or an alkali acetate, in particular sodium acetate. For cost reasons however only the latter can in practice be considered for production on an industrial scale, and even this known method is economically unsatisfactory, because relatively large amounts of catalyst (alkali acetate) have to be used which can only be recovered, if at all, at unjustifiably high cost. In addition, for acetylation of each hydroxyl group one mole of acetic anhydride is consumed and one mole of acetic acid produced which represents a by-product unusable in the process ie a waste product, since its reaction with ketene to yield acetic anhydride, while per se possible, is too expensive.

It has been an object of this invention, therefore, to provide an improved method of acetylation with acetic anhydride which avoids the disadvantages of the prior art and allows a more economical production of peracetylated sugar alcohols because it requires less catalyst and acetic anhydride and generates less acetic acid by-product which cannot be utilised in the process.

This object is accomplished by the process of the invention starting from the discovery that sugar alcohols may, by heating with acetic acid, which forms as a by-product during peracetylation with acetic anhydride and inevitably accumulates as a by-product during processing of the crude reaction product mixture, be smoothly and quickly reacted to give a partial acetylation product for example having an average of two acetyl radicals in the molecule. This is surprisingly accomplished, in contrast to the acetylation of sugars described in EP patent Application 0 091 159, without any use of catalyst. This partial acetylation product may, after distilling off the reaction water and any excess acetic acid be peracetylated by heating with acetic anhydride in stoichiometric or relatively slightly larger amounts in the presence of alkali acetate catalyst, amounts drastically reduced as compared to the prior art. The peracetylated sugar alcohol may be recovered from the reaction product by distilling off acetic acid that has formed as a by-product as well as any excess acetic anhydride, blending with water, cooling and separating the suspension obtained, e.g. by centrifuging or filtering. The peracetylated sugar alcohols obtained by the process have good crystalline form, fully satisfactory purity and, referred to the sugar alcohol used, are obtained in very high yields, and, as has surprisingly been found, are excellent activators not only for special percarbonates but for practically all commonly used bleaching agents of the oxygen type and in particular perborates.

Accordingly, the invention comprises a process for producing peracetylated products from sugar alcohols having at least four carbon atoms by reacting the sugar alcohol with acetic anhydride at an elevated temperature in the presence of an alkali catalyst, characterised in that the acetylation takes place in two steps, the first step comprising partial acetylation of the sugar alcohol by heating with more than two moles of acetic acid per mole of sugar alcohol in the absence of a catalyst and separating the reaction water formed and unreacted, excess acetic acid from the reaction product mixture, and the second step comprising completing the peracetylation of the partially acetylated sugar alcohol obtained in the first step by heating with at least one mole of acetic anhydride per mole free hydroxyl group in the presence of alkali acetate as catalyst, whereupon crystalline peracetylated sugar alcohol is obtained from the reaction product mixture by distilling off the acetic acid formed during the reaction and any unreacted acetic anhydride blending the reaction product mixture with water and cooling down, the crystalline peracetylated sugar alcohol being separated from the suspension in aqueous alkali acetate solution.

The process of the invention offers a number of surprising advantages over the state of the art and, in particular, allows a reduction in the consumption of acetic anhydride by around 30 to 80% and that of alkali acetate (catalyst) by about 50 to 80% and the concomitant production of acetic acid, which in the given context cannot be appropriately utilised, by around 40 to 90% without lowering the product quality or the yield referred to the sugar alcohol used.

The partially acetylated product produced in the first step of the process has preferably an average of two acetate groups per mole of sugar alcohol.

The molar ratio of acetic acid to sugar alcohol in the first step has to be larger than 2 and, considering that extremely high excesses do not result in any appreciably higher improvement of the course of reaction, is preferably selected within a range of 2.2 to 6, more preferably 2.5 to 5, and most preferably about 2.8 to 3.2.

The reaction temperature in the first step of the process of the invention may in principle be varied over a wide range, temperatures from 75 to 150, preferably 85 to 130, and most preferably 90 to 110°C having as a rule proved to be particularly suitable.

The reaction times required for a sufficiently complete reaction in the first step depend of course on the circumstances of the case and are preferably selected within a range of 0.25 to 4, more preferably 0.5 to 2, and most preferably about 0.75 to 1.75 hours.

In the second step of the process of the invention it is frequently sufficient to use acetic anhydride in the stoichiometric ratio, i.e. 1 mole per mole free hydroxyl group, to achieve complete peracetylation. It has however proved advantageous to use a certain excess of acetic anhydride which may be recovered without any tangible extra cost while the reaction product mixture is being distilled off. Acetic anhydride is therefore in the second step of the process of the invention preferably used at a ratio of 1.05 to 1.5, more preferably 1.08 to 1.25, and most preferably 1.1 to 1.15 moles per mole free hydroxyl group in the partially acetylated sugar alcohol.

The amount of catalyst to be employed in the second step of the process of the invention is relatively small and may vary over a wide range, it having been found advantageous to use per mole of partially acetylated sugar alcohol 0.1 to 1.5, more preferably 0.15 to 1, and most preferably 0.2 to 0.5 moles of alkali acetate, in particular sodium acetate.

The reaction times in the second step of the process of the invention may, by an appropriate selection of the other process conditions, be varied within wide limits and are preferably selected in a range of 0.15 to 3.5, more preferably 0.2 to 2.5, and most preferably 0.25 to 2 hours.

The reaction temperature, too, may in the second step in principle be selected within a wide range, but it has proved to be particularly suitable and advantageous to operate under reflux conditions.

A preferred embodiment of the invention provides a method of using as acetylating agent for the first step at least in part acetic acid which has been recovered and/or obtained as a by-product during the separation of the crude reaction product mixture of the first and/or, preferably, second step of the process of the invention by distillation.

The amount of by-product acetic acid of the process which cannot be economically used and, thus, the manufacturing costs of the peracetylated sugar alcohols may in the process of the invention be advantageously further reduced in that excess acetic acid from the process may be converted into an alkali-, in particular sodium-, acetate and in this form used as catalyst in the second step.

Preferred starting materials for the process of the invention are pentitols and hexitols, the sugar alcohols xylitol and sorbitol in particular, which are available in large quantities and at relatively low cost, being preferred for economic and technical reasons.

It is further noted with regard to the sugar alcohol starting material that for the process of the invention it is possible to use not only pure sugar alcohols, but also sugar alcohol mixtures, such as hydrogenated starch hydrolysates or technical xylitol or sorbitol from greens of xylitol and sorbitol crystallisation respectively, which may contain impurities such as non-reducing sugars, oligosaccharides and/or di-, tri- and/or higher molecular weight oligomeric sugar alcohols.

The example and the comparison test illustrate the invention and its chief advantages over the state of the art by reference to the production of hexaacetyl sorbitol.

## Example

100 kg of sorbitol was heated together with 99 kg of acetic acid for about 1 hour to 100°C, whereupon the reaction water and non-reacted acetic acid (33 kg) were distilled off. The intermediate product thus obtained was mixed with 25 kg of sodium acetate and 225 kg of acetic anhydride and brought to boiling point for 1.25 hours under reflux, whereupon the acetic acid (122 kg) formed during the reaction was distilled off. Water was next stirred into the distillation residue, the mixture cooled to 20°C and the white precipitate deposited during cooling removed from the suspension by filtering and then dried. In this manner 238kg of hexaacetyl sorbitol was obtained and as by-product 122 kg of acetic acid, of which 66 kg was reusable as acetylating agent in the first step of the next batch.

## Comparison Test

In a comparison test, 100 kg of sorbitol was mixed with 82 kg of sodium acetate and 336 kg of acetic anhydride and the mixture brought to boiling point for 2 hours under reflux.

The reaction product mixture thus obtained was then processed as in the aforementioned example, yielding 238 kg of hexaacetyl sorbitol and, as by-product, 197 kg of (not reusable) acetic acid.

## Claims

1. A process for producing peracetylated products from sugar alcohols having at least four carbon atoms by reacting the sugar alcohol with acetic anhydride at an elevated temperature in the presence of an alkali catalyst, characterised in that the acetylation takes place in two steps, the first step comprising partial acetylation of the sugar alcohol by heating with more than two moles of acetic acid per mole of sugar alcohol in the absence of a catalyst and separating the reaction water formed and unreacted, excess acetic acid from the reaction product mixture, and the second step comprising completing the peracetylation of the partially acetylated sugar alcohol obtained in the first step by heating with at least one mole of acetic anhydride per mole free hydroxyl group in the presence of alkali acetate as catalyst, whereupon crystalline peracetylated sugar alcohol is obtained from the reaction product mixture by distilling off the acetic acid formed during the reaction and any unreacted acetic anhydride blending the reaction product mixture with water and cooling down, the crystalline peracetylated sugar alcohol being separated from the suspension in aqueous alkali acetate solution formed in the process.

2. The process of Claim 1, characterised in that the partially acetylated sugar alcohol has an average of two acetate groups per mole of sugar alcohol.

3. The process of Claim 1 or Claim 2, characterised in that in the first step 2.2 to 6, preferably 2.5 to 5, and more preferably 2.8 to 3.2 moles of acetic acid are used per mole of sugar alcohol.

4. The process of any one of Claims 1 to 3, characterised in that the acetylation reaction in the first step is carried out at a temperature of 75 to 150, preferably 85 to 130, and most preferably 90 to 110° C.

5. The process of any one of Claims 1 to 4, characterised in that the acetylation in the first step is carried out for a reaction time of 0.25 to 4, preferably 0.5 to 2, and more preferably 0.75 to 1.75 hours.

6. The process of any one of Claims 1 to 5, characterised in that in the second step 1.05 to 1.5, preferably 1.08 to 1.25, and more preferably 1.1 to 1.15 moles of acetic anhydride is used per mole free hydroxyl group in the partially acetylated sugar alcohol.

7. The process of any one of Claims 1 to 6, characterised in that in the second step alkali acetate is used as catalyst in an amount per mole of partially acetylated sugar alcohol of 0.1 to 1.5, preferably 0.15 to 1, and more preferably 0.2 to 0.5 moles.

8. The process of any one of Claims 1 to 7, characterised in that in the second step the reaction time is 0.15 to 3.5, preferably 0.2 to 2.5, and more preferably 0.25 to 2 hours.

9. The process of any one of Claims 1 to 8, characterised in that the reaction in the second step is carried out under reflux conditions.

10. The process of any one of Claims 1 to 9, characterised in that as the acetylating agent in the first step acetic acid, is used which has been recovered by distillation from the reaction product mixture of the first step, and/or has been separated by distillation of acetic acid from the reaction product mixture of the second step.

11. The process of any one of Claims 1 to 10, characterised in that alkali acetate obtained from excess by-product acetic acid of the process is used as catalyst in the second step.

12. The process of any one of Claims 1 to 11, characterised in that the sugar alcohol is a pentitol, in particular xylitol, or a hexitol, in particular sorbitol.

## Revendications

1. Procédé d'obtention de produits peracétylés à partir d'alcools de sucres ayant au moins 4 atomes de carbone par réaction de l'alcool de sucre avec l'anhydride acétique à une température élevée en présence d'un catalyseur alcalin, caractérisé en ce que l'acétylation a lieu en deux étapes, la première étape comprenant l'acétylation partielle de l'alcool de sucre par chauffage avec plus de 2 moles d'acide acétique par mole d'alcool de sucre en l'absence d'un catalyseur et séparation du mélange de produits réactionnels de l'eau de réaction formée et de l'acide acétique en excès n'ayant pas réagi, et la seconde étape comprenant l'achèvement de la peracétylation de l'alcool de sucre partiellement acétylé obtenu dans la première étape par chauffage d'au moins une mole d'anhydride acétique par mole de groupe hydroxyle libre en présence d'acétate alcalin comme catalyseur, après quoi de l'alcool de sucre peracétylé cristallin est séparé du mélange de produits réactionnels par élimination par distillation de l'acide acétique formé au cours de la réaction et de tout anhydride acétique n'ayant pas réagi, addition d'eau au mélange de produits réactionnels et refroidissement, l'alcool de sucre peracétylé cristallin étant séparé de la suspension dans la solution aqueuse d'acétate alcalin formée dans le procédé.

2. Procédé suivant la revendication 1, caractérisé en ce que l'alcool de sucre partiellement acétylé comporte une moyenne de deux groupes acétate par mole d'alcool de sucre.

3. Procédé suivant la revendication 1 ou la revendication 2, caractérisé en ce que dans la première étape, on utilise 2,2 à 6, mieux encore 2,5 à 5 et notamment 2,8 à 3,2 moles d'acide acétique par mole d'alcool de sucre.

4. Procédé suivant l'une quelconque des revendications 1 à 3, caractérisé en ce que la réaction d'acétylation dans la première étape est conduite à une température de 75 à 150°C, mieux encore de 85 à 130°C et notamment de 90 à 110°C.

5. Procédé suivant l'une quelconque des revendications 1 à 4, caractérisé en ce que l'acétylation dans la première étape est conduite pendant une durée de réaction de 0,25 à 4 heures, mieux encore de 0,5 à 2 heures et notamment de 0,75 à 1,75 heure.

6. Procédé selon l'une quelconque des revendications 1 à 5, caractérisé en ce que dans la seconde étape, on utilise 1,05 à 1,5, mieux encore 1,08 à 1,25 et notamment 1,1 à 1,15 mole d'anhydride acétique par mole de groupe hydroxyle libre dans l'alcool de sucre partiellement acétylé.

7. Procédé suivant l'une quelconque des revendications 1 à 6, caractérisé en ce que dans la seconde étape, on utilise un acétate alcalin comme catalyseur en une quantité, par mole d'alcool de sucre partiellement acétylé, de 0,1 à 1,5, de préférence de 0,15 à 1 et notamment de 0,2 à 0,5 mole.

8. Procédé suivant l'une quelconque des revendications 1 à 7, caractérisé en ce que dans la seconde étape, la durée de réaction est de 0,15 à 3,5 heures, de préférence de 0,2 à 2,5 heures et notamment de 0,25 à 2 heures.

9. Procédé suivant l'une quelconque des revendications 1 à 8, caractérisé en ce que la réaction dans la seconde étape est conduite dans des conditions de reflux.

10. Procédé suivant l'une quelconque des revendications 1 à 9, caractérisé en ce qu'on utilise comme agent d'acétylation dans la première étape de l'acide acétique qui a été récupéré par distillation du mélange de produits réactionnels de la première étape, et/ou qui a été séparé par distillation d'acide acétique du mélange de produits réactionnels de la seconde étape.

11. Procédé suivant l'une quelconque des revendications 1 à 10, caractérisé en ce qu'on utilise comme catalyseur dans la seconde étape un acétate alcalin obtenu à partir de l'acide acétique en excès constituant un sousproduit.

12. Procédé suivant l'une quelconque des revendications 1 à 11, caractérisé en ce que l'alcool de sucre est un pentitol, en particulier le xylitol, ou un hexitol, en particulier le sorbitol.

**Patentansprüche**

1. Verfahren zur Herstellung peracetylierter Produkte aus Zuckeralkoholen, welche mindestens 4 Kohlenstoffatome enthalten, durch Umsetzung des Zuckeralkohols mit Essigsäure bei einer erhöhten Temperatur in Gegenwart eines Alkalikatalysators, dadurch gekennzeichnet, dass die Acetylierung in zwei Stufen stattfindet, wobei die erste Stufe die partielle Acetylierung des Zuckeralkohols durch Erhitzen mit mehr als 2 Mol Essigsäure pro Mol Zuckeralkohol in Abwesenheit eines Katalysators und Abtrennen des gebildeten Reaktionswassers und nicht-umgesetzter, überschüssiger Essigsäure aus dem Reaktionsproduktgemisch umfasst, und die zweite Stufe die Vollendung der Peracetylierung des in der ersten Stufe erhaltenen partiell acetylierten Zuckeralkohols durch Erhitzen mit mindestens einem Mol Essigsäureanhydrid pro Mol freier Hydroxylgruppe in Gegenwart von Alkaliacetat als Katalysator umfasst, worauf kristallierter peracetylierter Zuk-

keralkohol aus dem Reaktionsproduktgemisch erhalten wird durch Abdestillieren der während der Reaktion gebildeten Essigsäure und jeglichem nicht-umgesetzten Essigsäureanhydrid, Vermischen des Reaktionsproduktgemisches mit Wasser und Abkühlen erhalten wird, wobei der kristalline peracetylierte Zuckeralkohol aus der während des Verfahrens gebildeten Suspension in wässeriger Alkaliacetatlösung abgetrennt wird.

2. Verfahren nach Anspruch 1, dadurch gekennzeichnet, dass der partiell acetylierte Zuckeralkohol einen Durchschnitt von zwei Acetatgruppen pro Mol Zuckeralkohol aufweist.

3. Verfahren nach Anspruch 1 oder Anspruch 2, dadurch gekennzeichnet, dass in der ersten Stufe 2,2 bis 6, vorzugsweise 2,5 bis 5 und mehr bevorzugt 2,8 bis 3,2 Mol Essigsäure pro Mol Zuckeralkohol verwendet werden.

4. Verfahren nach einem der Ansprüche 1 bis 3, dadurch gekennzeichnet, dass die Acetylierungsreaktion in der ersten Stufe bei einer Temperatur von 75 bis 150, vorzugsweise 85 bis 130 und am meisten bevorzugt 90 bis 110°C durchgeführt wird.

5. Verfahren nach einem der Ansprüche 1 bis 4, dadurch gekennzeichnet, dass die Acetylierung in der ersten Stufe während einer Reaktionszeit von 0,25 bis 4, vorzugsweise 0,5 bis 2 und mehr bevorzugt 0,75 bis 1,75 Stunden durchgeführt wird.

6. Verfahren nach einem der Ansprüche 1 bis 5, dadurch gekennzeichnet, dass in der zweiten Stufe 1,05 bis 1,5, vorzugsweise 1,08 bis 1,25 und mehr bevorzugt 1,1 bis 1,15 Mol Essigsäureanhydrid pro Mol freie Hydroxylgruppe in partiell acetyliertem Zuckeralkohol verwendet werden.

7. Verfahren nach einem der Ansprüche 1 bis 6, dadurch gekennzeichnet, dass in der zweiten Stufe Alkaliacetat als Katalysator in einer Menge pro Mol partiell acetyliertem Zuckeralkohol von 0,1 bis 1,5, vorzugsweise 0,15 bis 1 und mehr bevorzugt 0,2 bis 0,5 Mol verwendet wird.

8. Verfahren nach einem der Ansprüche 1 bis 7, dadurch gekenn zeichnet, dass in der zweiten Stufe die Reaktionszeit 0,15 bis 3,5, vorzugsweise 0,2 bis 2,5 und mehr bevorzugt 0,25 bis 2 Stunden beträgt.

9. Verfahren nach einem der Ansprüche 1 bis 8, dadurch gekennzeichnet, dass die Reaktion in der zweiten Stufe unter Rückflussbedingungen durchgeführt wird.

10. Verfahren nach einem der Ansprüche 1 bis 9, dadurch gekennzeichnet, dass die als Acetylierungsmittel in der ersten Stufe Essigsäure verwendet wird, welche durch Destillation aus dem Reaktionsproduktgemisch der ersten Stufe zurückgewonnen wurde und/oder durch Destillation von Essigsäure aus dem Reaktionsproduktgemisch der zweiten Stufe abgetrennt wurde.

11. Verfahren nach einem der Ansprüche 1 bis 10, dadurch gekennzeichnet, dass Alkaliacetat, welches aus überschüssiger Nebenprodukt-Essigsäure des Verfahrens erhalten wurde, als Katalysator in der zweiten Stufe verwendet wird.

12. Verfahren nach einem der Ansprüche 1 bis 11, dadurch gekennzeichnet, dass der Zuckeralkohol ein Pentit ist, insbesondere Xylit, oder ein Hexit, insbesondere Sorbit.